(19) Europäisches Patentamt / European Patent Office / Office européen des brevets

(11) **EP 2 854 835 B1**

(12) **FASCICULE DE BREVET EUROPEEN**

(45) Date de publication et mention
de la délivrance du brevet:
**04.04.2018 Bulletin 2018/14**

(21) Numéro de dépôt: **13730039.8**

(22) Date de dépôt: **05.06.2013**

(51) Int Cl.:
*A61K 38/17* *(2006.01)*    *A61K 9/19* *(2006.01)*
*A61K 38/42* *(2006.01)*    *A61K 47/22* *(2006.01)*
*A61K 47/26* *(2006.01)*    *A61K 35/62* *(2006.01)*

(86) Numéro de dépôt international:
**PCT/FR2013/051271**

(87) Numéro de publication internationale:
**WO 2013/182806 (12.12.2013 Gazette 2013/50)**

(54) **PROCÉDÉ DE LYOPHILISATION D'HÉMOGLOBINE D'ANNÉLIDES**

RINGELWURM-HÄMOGLOBIN-LYOPHILISIERUNGSVERFAHREN

ANNELID HAEMOGLOBIN LYOPHILISATION PROCESS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **05.06.2012 FR 1255204**

(43) Date de publication de la demande:
**08.04.2015 Bulletin 2015/15**

(73) Titulaire: **Hemarina
29600 Morlaix (FR)**

(72) Inventeurs:
• **ZAL, Franck
F-29600 Ploujean-Morlaix (FR)**
• **ROUSSELOT, Morgane
F-29250 Saint Pol de Léon (FR)**

(74) Mandataire: **Lavoix
2, place d'Estienne d'Orves
75441 Paris Cedex 09 (FR)**

(56) Documents cités:
WO-A1-2007/035583    WO-A1-2010/128159
WO-A1-2013/001196    WO-A2-03/026786
WO-A2-2009/007532    WO-A2-2009/050343
CN-A- 101 642 565

• J.R HARRIS ET AL: "Transmission electron microscopical studies on some haemolymph proteins from the marine polychaete Nereis virens", MICRON, vol. 32, no. 6, août 2001 (2001-08), pages 599-613, XP055076677, ISSN: 0968-4328, DOI: 10.1016/S0968-4328(00)00051-2

• JOHNSTON D S ET AL: "The influence of sugars on the properties of freeze-dried lysozyme and haemoglobin", THERMOCHIMICA ACTA, ELSEVIER SCIENCE PUBLISHERS, AMSTERDAM, NL, vol. 144, no. 2, 10 juin 1989 (1989-06-10) , pages 195-208, XP026579699, ISSN: 0040-6031, DOI: 10.1016/0040-6031(89)85100-7 [extrait le 1989-06-10]

• P LABRUDE ET AL: "Influence de l'addition d'albumine sur la conservation de la solution d'hémoglobine lyophilisée en présence de glucose", REVUE FRANCAISE DE TRANSFUSION ET IMMUNO-HÉMATOLOGIE, vol. 23, no. 1, 1980, pages 23-34, XP055050746, ISSN: 0338-4535, DOI: 10.1016/S0338-4535(80)80103-6

**Description**

La présente invention a trait à un procédé de lyophilisation d'au moins une globine, un protomère de globine ou une hémoglobine extracellulaire d'Annélides, ainsi qu'au lyophilisat obtenu.

L'hémoglobine extracellulaire d'Annélides, est utile en tant que substitut sanguin, et permet de traiter des problèmes liés à une déficience en oxygène.

Par substitut sanguin, on entend tout produit ou solution permettant de compenser une perte sanguine consécutive à une hémorragie en apportant des transporteurs d'oxygène. Un substitut sanguin est différent d'un sang artificiel, car le substitut sanguin ne peut pas remplir toutes les fonctions assurées par le sang, telles que le transport d'hormones par exemple.

Actuellement, deux classes pharmaceutiques de produits de substitution sont déjà connues : il s'agit des Perfluorocarbones (PFC) et des « Hemoglobin Oxygen Carrier (HBOC) », ou transporteurs d'oxygène à base d'hémoglobine. Les PFC sont des composés synthétisés chimiquement permettant le transport d'$O_2$ sous forme dissoute dans la circulation sanguine et jusqu'aux tissus.

Les HBOC proviennent de la purification puis modification chimique d'Hb bovine ou humaine (ces dernières provenant de poches de sang périmées) ou sont encore issus de la synthèse par génie génétique.

Afin de stocker les HBOC, une méthode classique consiste à conditionner les molécules d'hémoglobine en solution. Afin de garantir une meilleure stabilité, dans certains cas, il est nécessaire de les conserver entre -20°C et -80°C avant leur utilisation. Leur transport est alors réalisé sous carboglace pour s'assurer de l'absence de décongélation. Ces différentes conditions impliquent un coût supplémentaire et une logistique de stockage et de transport plus contraignante qui peuvent s'avérer limitants pour certaines applications.

WO2010/128159 décrit l'hémoglobine de *Nereididae*, WO2007/035583 décrit la lyophilisation d'hémoglobine de vertébrés, et WO03/026786 décrit un procédé de lyophilisation qui utilise un composé diminuant la teneur en solvant résiduel.

Il existe donc un besoin de disposer d'une hémoglobine fonctionnelle qui soit facilement stockée et transportée, y compris dans des environnements à distance sans réfrigération.

Des solutions existent, et l'une d'entre elles est la lyophilisation.

Cependant, des études préliminaires ont révélé que les molécules d'hémoglobine sont en partie dégradées par le processus de lyophilisation, ce qui altère en particulier leur fonctionnalité, à savoir leur capacité à fixer réversiblement l'oxygène. Ceci est d'ailleurs décrit dans la littérature pour les protéines de manière générale (HELLER.Martin.C, CARPENTER.John.F, RANDOLPH.Theodore.W, "Protein Formulation and Lyophilization Cycle Design : Prevention of Damage due to Freeze-Concentration Induced Phase Separation" Biotechnology and Bioengineering, Vol 63, N°2, 1999.).

Les inventeurs ont maintenant découvert que, de façon surprenante, l'hémoglobine extracellulaire d'Annélides, lorsqu'elle est mélangée à un agent stabilisant spécifique, peut être lyophilisée, tout en conservant sa structure quaternaire, sa fonctionnalité et son efficacité.

La présente invention concerne ainsi un procédé de lyophilisation d'au moins une hémoglobine extracellulaire d'Annélides. La présente invention se rapporte également au lyophilisat ainsi obtenu. Ce dernier permet en effet de stocker l'hémoglobine extracellulaire d'Annélides, sous un format économique, pratique (i.e. il nécessite un minimum de place), tout en conservant les propriétés structurelles et fonctionnelles de l'hémoglobine.

Le lyophilisat selon l'invention comprend au moins une hémoglobine extracellulaire de la famille des *Arenicolidae* ou de la famille des *Nereididae*, et un agent stabilisant choisi parmi les disaccharides.

Il est également décrit une composition comprenant :

- une solution comprenant au moins une globine, un protomère de globine ou une hémoglobine extracellulaire d'Annélides, et
- un agent stabilisant choisi parmi les disaccharides, les polyols et les antioxydants, de préférence choisi parmi le tréhalose et l'acide ascorbique.

L'hémoglobine extracellulaire d'Annélides est présente chez les trois classes d'Annélides : les Polychètes, les Oligochètes et les Achètes. On parle d'hémoglobine extracellulaire car elle est naturellement non contenue dans une cellule, et peut donc circuler librement dans le système sanguin sans modification chimique pour la stabiliser ou la rendre fonctionnelle.

L'hémoglobine extracellulaire d'Annélides est un biopolymère géant de poids moléculaire compris entre 2000 et 4000 kDa, constitué d'environ 200 chaînes polypeptidiques comprises entre 4 et 12 types différents que l'on regroupe généralement en deux catégories.

La première catégorie, comptant 144 à 192 éléments, regroupe les chaînes polypeptidiques dites "fonctionnelles" qui portent un site actif de type hème, et sont capables de lier réversiblement l'oxygène ; ce sont des chaînes de type globine dont les masses sont comprises entre 15 et 18 kDa et qui sont très similaires aux chaînes de type α et β de vertébrés. La deuxième catégorie, comptant 36 à 42 éléments, regroupe les chaînes polypeptidiques dites de "structure" ou « linkers » possédant peu ou pas de site actif mais permettant l'assemblage des sous-unités appelées douzièmes ou protomères. Chaque molécule d'hémoglobine est constituée de deux hexagones superposés que l'on a nommés bicouche hexagonale (hexagonal bilayer) et chaque hexagone est lui-même formé par l'assemblage de six sous-unités (ou "douzièmes" ou « protomères ») en forme de goutte d'eau. La molécule native est formée de douze de ces sous-unités (dodécamère ou protomère). Chaque sous-unité a une masse moléculaire comprise entre 200 et 250 kDa, et constitue l'unité fonctionnelle de la molécule native.

De préférence, l'hémoglobine extracellulaire d'Annélides est choisie parmi les hémoglobines extracellulaires d'Annélides Polychètes, de préférence parmi les hémoglobines extracellulaires de la famille des *Arenicolidae* et les hémoglobines extracellulaires de la famille des *Nereididae.* Encore plus préférentiellement, l'hémoglobine extracellulaire d'Annélides est choisie parmi l'hémoglobine extracellulaire d'*Arenicola marina* et l'hémoglobine extracellulaire de *Nereis,* plus préférentiellement l'hémoglobine extracellulaire d'*Arenicola marina.*

Il est également décrit un lyophilisat ou une composition comprenant au moins un protomère de globine de l'hémoglobine extracellulaire d'Annélides. Ledit protomère constitue l'unité fonctionnelle de l'hémoglobine native, comme indiqué ci-dessus. Enfin, il est également décrit un lyophilisat ou une composition comprenant au moins une chaîne de globine de l'hémoglobine extracellulaire d'Annélides. Une telle chaîne de globine peut notamment être choisie parmi les chaînes de globine de type Ax et/ou Bx d'hémoglobine extracellulaire d'Annélides.

L'hémoglobine extracellulaire d'Annélides et ses protomères de globine ont une activité superoxyde dismutase (SOD) intrinsèque, et la présence d'antioxydant n'est pas nécessaire pour leur fonctionnement, contrairement à l'utilisation d'une hémoglobine de mammifères, pour laquelle les molécules antioxydantes sont contenues à l'intérieur du globule rouge et ne sont pas liées à l'hémoglobine. D'autre part, l'hémoglobine extracellulaire d'Annélides, ses protomères de globine et/ou ses globines ne nécessitent pas de cofacteur pour fonctionner, contrairement à l'hémoglobine de mammifère, notamment humaine. Enfin, l'hémoglobine extracellulaire d'Annélides, ses protomères de globine et/ou ses globines ne possédant pas de typage sanguin, ils permettent d'éviter tout problème de réaction immunologique.

L'hémoglobine extracellulaire d'Annélides, ses protomères de globine et/ou ses globines peuvent être natifs ou recombinants.

La composition décrite comprend également une solution. Cette solution est capable de créer un environnement salin adéquat pour l'hémoglobine, ses protomères et ses globines, et permet ainsi le maintien de la structure quaternaire, et donc de la fonctionnalité de cette molécule. Grâce à la solution, l'hémoglobine, ses protomères et ses globines sont capables d'assurer leur fonction d'oxygénation.

La solution est une solution aqueuse comprenant des sels, de préférence des ions chlorure, sodium, calcium, magnésium et potassium, et confère à la composition un pH compris entre 6.5 et 7.8 ; sa formulation est similaire à celle d'un liquide physiologiquement injectable. Dans ces conditions, l'hémoglobine extracellulaire d'Annélides, ses protomères de globine et ses globines restent fonctionnels.

Dans la présente description, le pH s'entend à température ambiante (20±5°C), sauf mention contraire.

De préférence, la solution est une solution aqueuse comprenant du chlorure de sodium, du chlorure de calcium, du chlorure de magnésium, du chlorure de potassium, ainsi que du sodium gluconate et du sodium acétate, et a un pH compris entre 6.5 et 7.8, de préférence égal à 7,1 ± 0,5, de préférence d'environ 7.35. Plus préférentiellement, la solution de stabilisation est une solution aqueuse comprenant 0-100 mM de NaCl, de préférence 90 mM de NaCl, 23 Mm de Na-gluconate, 2,5 mM de CaCl$_2$, 27 mM de Na-acétate, 1,5 mM de MgCl$_2$, 5 mM de KCl, et a un pH de 7,1 ± 0,5, pouvant contenir entre 0 et 100 mM d'antioxydant de type acide ascorbique et/ou glutathion réduit. Ladite solution a de préférence une osmolarité comprise entre 300 et 450, de préférence entre 300 et 350, et préférentiellement de 302 mOsmol/L.

La composition décrite et le lyophilisat selon l'invention comprennent également un agent stabilisant. Cet agent stabilisant assure le maintien de la structure quaternaire et donc de la fonctionnalité de l'hémoglobine, ses globines et ses protomères, même après lyophilisation.

Par « agent stabilisant », on entend un disaccharide, un polyol, et/ou un antioxydant. Les disaccharides comprennent notamment le saccharose, le tréhalose et raffinose, de préférence le tréhalose. L'efficacité de l'agent stabilisant est déterminée en comparant la physico-chimie et les propriétés fonctionnelles de l'hémoglobine avant et après la lyophilisation.

L'agent stabilisant selon l'invention est choisi parmi les disaccharides.

De préférence, les disaccharides sont choisis parmi le tréhalose et le saccharose. Plus préférentiellement, le disaccharide est le tréhalose. De préférence, les polyols sont choisis parmi le mannitol et le sorbitol. Enfin, de préférence, l'antioxydant est l'acide ascorbique.

Le tréhalose est aussi appelé α-D-glucopyranosyl-α-D-glucopyranoside ou alpha,alpha-tréhalose, ou α-D-glucopyranosyl-α-D-glucopyranoside, dihydrate. C'est un disaccharide composé de deux molécules de glucose reliées entre elles par une liaison α,α-1,1 (ou «1,1-α-glycosidique ») particulièrement stable.

Le saccharose (aussi appelé « sucrose » dans la présente demande) est un disaccharide formé par la condensation d'une molécule de glucose avec une molécule de fructose. Son nom chimique est le β-D-fructofuranosyl-(2↔1)-α-D-glucopyranoside.

Le mannitol, ou 1,2,3,4,5,6-hexanehexol, et le sorbitol, ou (2R,3S,4S,5S)-Hexane-1,2,3,4,5,6-hexol, sont des polyols.

Enfin, l'acide ascorbique est un acide organique ayant des propriétés anti-oxydantes. Il peut être présent sous forme D ou L. De préférence, l'agent stabilisant est l'acide L-ascorbique, ou vitamine C.

De préférence, l'agent stabilisant est le tréhalose. De préférence, la composition et/ou le lyophilisat selon l'invention comprennent le tréhalose et l'acide ascorbique.

La composition décrite et le lyophilisat selon l'invention peuvent comprendre des sels. Ces sels peuvent être choisis parmi les sels de sodium, de calcium, de magnésium et de potassium. De préférence, les sels sont choisis parmi le chlorure de sodium, le chlorure de calcium, le chlorure de magnésium, le chlorure de potassium, le sodium gluconate et le sodium acétate.

L'invention a également pour objet un procédé de préparation d'un lyophilisat, tel que décrit en revendication 5.

Il est également décrit un procédé de préparation d'un lyophilisat, comprenant :

i) le mélange d'une solution comprenant au moins une globine, un protomère de globine ou une hémoglobine extracellulaire d'Annélides avec un agent stabilisant choisi parmi les disaccharides, les polyols et les antioxydants,

ii) la congélation du mélange obtenu en i) à une température comprise entre -10°C et - 100°C, de préférence entre -20°C et -100°C pendant un temps d'au moins 24h, de préférence au moins 48h ;

iii) la sublimation du mélange congelé obtenu en ii) pendant au moins 2h, sous vide ;

iv) le séchage final du mélange obtenu en iii), jusqu'à l'obtention d'une poudre.

Le mélange de l'étape i) se fait notamment par vortex.

De préférence, l'agent stabilisant, notamment le disaccharide ou le polyol, est présent dans le mélange de l'étape i) du procédé selon l'invention en une concentration comprise entre 1 et 500 mg/ml. Alternativement, l'agent stabilisant, notamment l'antixoydant, est présent dans le mélange de l'étape i) en une concentration comprise entre 3 et 20 mM. Plus préférentiellement encore, l'agent stabilisant présent dans le mélange de l'étape i) est choisi parmi :

- le tréhalose et le saccharose, présents en une concentration comprise entre 50 et 70 mg/ml, de préférence d'environ 55 mg/ml ou 65 mg/ml,
- le tréhalose et le saccharose, présents en une concentration d'environ 100 mg/ml,
- le mannitol présent en une concentration comprise entre 50 et 100 g/l, et
- l'acide ascorbique présent en une concentration d'environ 5 mM.

La solution obtenue en fin d'étape i) comprend ainsi au moins une globine, un protomère de globine ou une hémoglobine extracellulaire d'Annélides, et l'agent stabilisant.

Cette solution est soumise à une lyophilisation. Le cycle de lyophilisation peut comprendre trois étapes :

- la congélation (étape ii) du procédé selon l'invention) :

Cette première phase consiste à congeler la solution de telle façon que l'eau contenue soit transformée en glace.

De préférence, la congélation de l'étape ii) du procédé selon l'invention est effectuée à une température comprise entre -10°C et -100°C, de préférence entre -20°C et -90°C pendant au moins 24h, de préférence au moins 48h. De préférence, la congélation est effectuée à environ -20°C pendant au moins 24h, de préférence au moins 48h.

- la dessiccation primaire ou sublimation (étape iii) du procédé selon l'invention) :

L'étape de sublimation permet le passage de la glace présente dans la solution congelée de l'état solide à l'état gazeux, sans étape intermédiaire. La solution congelée est desséchée du fait d'une mise sous vide ; la glace devient alors de la vapeur.

La sublimation se fait à l'aide d'une pompe à vide poussé, d'une pompe mécanique ou d'une cryo-pompe.

De préférence, la sublimation de l'étape iii) est effectuée pendant au moins 4h.

- la dessiccation secondaire ou séchage final (étape iv) du procédé selon l'invention) :

Lorsque la glace est totalement sublimée, la phase de dessiccation secondaire peut débuter. Elle permet d'extraire par désorption les molécules d'eau piégées à la surface des produits séchés.

A la fin de la lyophilisation, et donc du procédé, le lyophilisat obtenu comprend entre 0,1 et 5% en poids d'eau. Le lyophilisat est une poudre, qui se redissout complètement dans un liquide hydrophile, sans résidus insolubles. La poudre peut être stockée dans des bouteilles ou des flacons de verre ou de plastique, de préférence en verre.

L'hémoglobine lyophilisée ainsi obtenue est facile à transporter et à stocker. Le lyophilisat selon la présente invention est ainsi facile à reconstituer et est prêt à être utilisé.

Il est également décrit une composition comprenant le lyophilisat selon l'invention, et un diluant. Le lyophilisat peut en effet être dilué au moment opportun avec un diluant, afin de restituer la solution d'hémoglobine initiale. De préférence, le diluant est de l'eau ultrapure, afin de ne pas modifier la concentration des sels de la solution, et d'obtenir une composition ayant un volume équivalent à celui d'avant la lyophilisation.

L'invention est décrite plus en détail dans les exemples suivants. Ces exemples sont fournis à des fins d'illustration uniquement, et ne sont pas limitatifs.

## Exemple 1

**Matériel et méthodes**

Le protocole d'étude

Les inventeurs ont évalué les effets de la lyophilisation et l'impact des excipients ajoutés à la formulation sur :

- la structure quaternaire des hémoglobines,
- la fonctionnalité : capacité à fixer l'oxygène,
- l'efficacité des hémoglobines dans des modèles cellulaires.

La préparation des hémoglobines (Hb)

Pour l'ensemble des études, le même lot d'Hb a été utilisé. Une quantité calculée d'Hb est mise à décongeler à $5 \pm 3$°C pendant 1 heure. Une fois décongelée, la quantité nécessaire d'excipient est rajoutée sous forme liquide pour atteindre la concentration désirée.

La solution ainsi obtenue est congelée à -80°C pendant au moins 24h. La solution congelée est ensuite lyophilisée dans le lyophilisateur pendant environ 4h jusqu'à obtention d'une poudre. Cette poudre est ensuite conservée à -80°C avant l'étude de stabilité.

La reconstitution est réalisée avec de l'eau ultrapure pour ne pas modifier la concentration en sels et dans un volume équivalent à celui d'avant lyophilisation.

Les outils analytiques

L'étude de suivi du produit comporte trois phases : le suivi de la structure de la molécule, le suivi de sa fonctionnalité et enfin le suivi de la concentration en hémoglobine.

*Le suivi de la structure*

La structure est suivie par une méthode de chromatographie spécifique aux protéines : la FPLC (Fast Protein Liquid Chromatography). Elle utilise le principe de l'exclusion stérique. Le système utilisé est commercialisé par Dionex® sous le nom commercial Ultimate 3000. Il est entièrement automatisé et piloté par le logiciel accompagnateur Chromeleon ®.

Les colonnes utilisées sont commercialisées par GE Healthcare® et sont du type Superose 6, $10 \times 300$ mm ; elles permettent une séparation des molécules dont le poids moléculaire est compris entre 5 MDa et 5000 Da en une heure à un débit de 0,5 mL/min. La séparation se fait en condition isocratique c'est-à-dire qu'il n'y a qu'un seul tampon d'élution.

L'acquisition dans le cadre de l'étude de l'hémoglobine se fait à 2 longueurs d'onde (280 nm et 414 nm). Afin de détecter la molécule cible, l'hémoglobine, la densité optique est mesurée à 280 nm (pic d'absorption des protéines) et à 414 nm (pic d'absorption de l'hème). L'acquisition à 414 nm permet d'identifier l'hémoglobine parmi les autres protéines observées à 280 nm. A cette même longueur d'onde, il est aussi possible de suivre la cinétique de dissociation de l'hémoglobine. Le logiciel Chroméléon® fourni avec l'appareillage, permet à la fois de récolter les données mais également de les traiter.

Le traitement des données se fait par l'analyse des chromatogrammes obtenus à 280 nm et à 414 nm.

Le logiciel permet d'intégrer les chromatogrammes :

- L'aire sous courbe du pic d'intérêt est proportionnelle à la concentration ;
- Le pourcentage relatif de pureté de chaque pic calculé par le logiciel permet de suivre l'évolution de la dégradation de la molécule au cours du temps.

Dans le cas de l'étude, des cinétiques permettant de quantifier les effets des excipients sélectionnés et le comportement de la molécule sur une semaine sont réalisées. Pour cela, les chromatogrammes de chaque excipient sont intégrés à chaque point (du temps $T_0$ jusqu'au jour 5) puis les données générées par le logiciel (l'aire sous courbe et le pourcentage relatif de pureté) sont traitées graphiquement à l'aide du logiciel Graphpad® afin de déterminer les cinétiques de dégradation et de les comparer.

*Le suivi de la fonctionnalité*

La fonctionnalité de la molécule d'hémoglobine est définie par sa capacité à fixer réversiblement l'oxygène. Cette étude est possible avec la spectrophotométrie UV-visible.

L'hémoglobine possède une signature spectrophotométrique caractéristique qui évolue en fonction de sa fonctionnalité et de son état d'oxydation. La mesure s'effectue sur une fenêtre de longueurs d'onde comprise entre 250 et 700 nm. Ainsi, des spectres d'absorption différents selon l'état d'oxydation sont obtenus.

Par exemple, l'hémoglobine d'*Arenicola marina* (HbAm) présente à son état oxygéné deux pics dans le visible, i.e. les bandes alpha et bêta respectivement présentes à 576 nm et 540 nm, et une bande dite de Soret à 414 nm. Les premières citées sont caractéristiques de l'absorption du complexe formé entre le noyau d'hème, le fer et l'oxygène. La bande de Soret est quant à elle synonyme de l'absorption du complexe formé entre la chaîne polypeptidique et le noyau d'hème.

L'hémoglobine possède des propriétés spectrales caractéristiques de ses changements de conformation entre les états oxygénés et non oxygénés.

Ainsi, en fonction des maxima relevés, les différents états de l'hémoglobine peuvent être déterminés en nous reportant au tableau suivant :

| Bande de Soret | Bande Alpha | Bande Béta | Forme de l'hémoglobine |
|---|---|---|---|
| 414 ± 2 nm | 540 ± 2 nm | 576 ± 2 nm | Oxyhémoglobine |
| 430 ± 2 nm | 555 ± 2 nm | 555 ± 2 nm | Déoxyhémoglobine |
| 418 ± 2 nm | 535 ± 2 nm | 570 ± 2 nm | Carboxyhémoglobine |
| 406 ± 2 nm | 500 ± 2 nm | 630 ± 2 nm | Méthémoglobine |

Enfin, le pourcentage d'hémoglobine oxydée peut être déterminé. Les spectres UV-visible acquis entre 250 et 700 nm sont normalisés à 523 nm (point isobestique). Les absorbances à 576 nm (bande alpha) et 540 nm (bande béta) sont additionnées pour chaque spectre. De même les absorbances pour un échantillon de référence et idéalement ne présentant pas d'oxydation (dans le cas de la lyophilisation = le J0 d'un échantillon non lyophilisé est choisi comme référence) sont relevées. Puis la même opération est effectuée pour un échantillon considéré comme 100 % oxydé (obtenu par incubation à 37 °C jusqu'à disparition des bandes alpha et bêta).
Le pourcentage d'oxydation de la molécule est obtenu par le calcul suivant :

$$\% \text{ Hb oxydée} = 100 - \frac{[A\,Hb - A100] \times 100}{A0 - A\,100}$$

Avec A = $A_{540}$ + $A_{576}$ ; A Hb = $A_{540}$ + $A_{576}$ de l'échantillon d'hémoglobine ; A 0 = $A_{540}$ + $A_{576}$ du 0 % oxydé ; A 100 = $A_{540}$ + $A_{576}$ du 100 % oxydé.

De même que pour le suivi de la structure, les spectres sont superposés pour chaque condition afin de visualiser l'effet des excipients sur une semaine puis l'évolution des pourcentages d'oxydation est traitée graphiquement par le logiciel Graphpad® afin de déterminer la cinétique d'auto oxydation dans les différentes conditions testées.

*Le suivi de la concentration*

L'hémoglobine est dosée par spectrophotométrie à l'aide du réactif de Drabkin's qui permet de doser précisément l'hème, site actif de l'hémoglobine.

Les tests d'efficacité

Afin d'évaluer l'efficacité des hémoglobines d'*Arenicola marina* (HbAm) et de *Nereis* (HbN), deux modèles cellulaires ont été développés au laboratoire, chacun représentatif des applications pour lesquelles les molécules sont dédiées.

Pour HbAm, il s'agit d'un modèle cellulaire qui mime les conditions de conservation des organes en attente de transplantation. Un test de toxicité cellulaire est utilisé afin d'évaluer les lésions induites par la conservation à froid : ce test correspond au relargage de la Lactate Déshydrogénase (LDH). La LDH est une enzyme présente dans le cytosol des cellules et sa libération dans le surnageant de culture est le reflet d'une perméabilisation de la membrane plasmique et donc d'une mort cellulaire. L'efficacité des HbAm est évaluée en comparant le pourcentage de libération de la LDH dans les conditions de conservation d'organes avec et sans HbAm.

Le second modèle développé est basé sur l'application de la molécule HbN dans le cadre de la bioproduction de protéines recombinantes. La lignée cellulaire correspond à une lignée couramment utilisée pour la bioproduction. Les cellules sont ensemencées à une densité cellulaire déterminée en présence ou non d'une quantité connue de HbN. Après 4 jours de culture, la densité cellulaire et la viabilité cellulaire sont mesurées. L'efficacité des HbN est évaluée en comparant la croissance et la viabilité cellulaires dans les conditions de culture avec et sans HbN.

**Résultats pour HbAm**

Suivi de la stabilité de HbAm après reconstitution ($T_0$) *Suivi de la concentration en hémoglobine après reconstitution*

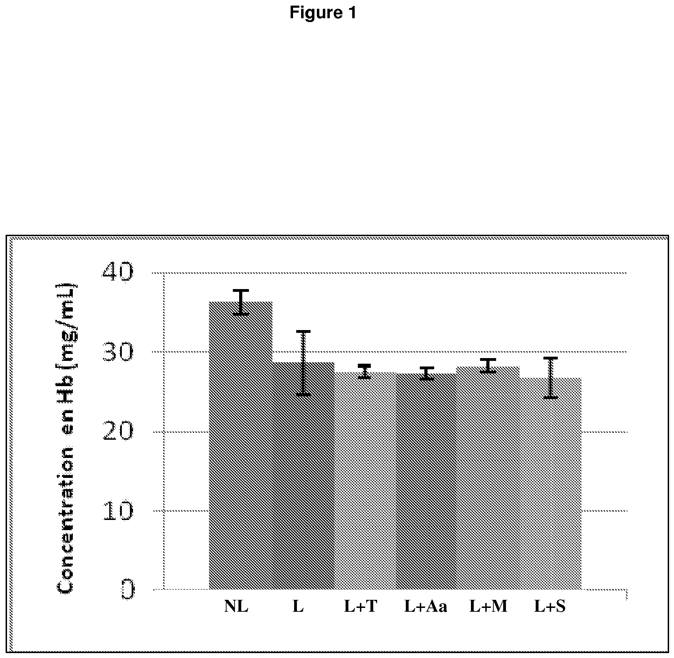

Les résultats du dosage d'HbAm après reconstitution suite à l'étape de lyophilisation indiquent une diminution de la concentration en Hb après reconstitution, quelles que soient les conditions de lyophilisation (voir Figure 1. Légende : NL = Non lyophilisée ; L = Lyophilisée ; L+T = Lyophilisée avec tréhalose ; L+Aa = Lyophilisée avec acide ascorbique ; L+M = Lyophilisée avec mannitol ; L+S = Lyophilisée avec sucrose). Cette perte de moins de 10 % indique que la lyophilisation diminue le pouvoir hydrophile de la molécule.

*Suivi de la stabilité structurale à T0*

Les pourcentages de pureté à 280nm et à 414 nm de HbAm sont similaires pour les différentes compositions testées.

Au vu des écarts-type, les résultats obtenus montrent qu'il n'y a pas de différence significative de pureté entre les différentes conditions testées. Ainsi à T0, après reconstitution, la structure quaternaire de l'HbAm n'est pas significativement affectée ni par la lyophilisation, ni par la présence des différents excipients.

Suivi de la fonctionnalité de HbAm reconstituée

Afin de juger au mieux de l'impact de la lyophilisation sur la fonctionnalité de la molécule après reconstitution ($T_0$), le pourcentage d'oxydation et les modifications de la signature spectro-photométrique de l'hémoglobine sont mesurés.

Le processus de lyophilisation provoque une oxydation de la molécule d'environ 20% par rapport à la molécule non lyophilisée. Tous les excipients testés, et en particulier l'acide ascorbique et le tréhalose, montrent un effet positif sur la protection de la molécule contre l'oxydation.

Le tréhalose et l'acide ascorbique ont permis à la fois de protéger la molécule contre l'oxydation induite par le processus de lyophilisation mais aussi de réduire la proportion de molécule oxydée initialement.

L'étude des spectres UV-Visible nous montre que les différentes conditions testées n'affectent pas de façon significative la fonctionnalité de HbAm après reconstitution : les bandes de Soret, alpha et béta sont présentes aux longueurs d'onde d'une hémoglobine fonctionnelle (cf tableau 1). Dans le cas de l'hémoglobine lyophilisée seule, bien que 20 % d'oxydation soit observé, la molécule reste globalement fonctionnelle.

| Conditions | Bande de Soret | Bande Alpha | Bande Béta | Fonctionnalité |
|---|---|---|---|---|
| HbAm NL | 414 nm | 540 nm | 574 nm | Oxyhémoglobine |
| HbAm L | 412 nm | 538 nm | 574 nm | |
| HbAm L + Tréhalose | 414 nm | 538 nm | 574 nm | |
| HbAm L + Mannitol | 412 nm | 538 nm | 574 nm | |
| HbAm L + Sucrose | 414 nm | 538 nm | 574 nm | |
| HbAm L + Acide ascorbique | 414 nm | 540 nm | 574 nm | |

Tableau 1: Longueurs d'onde relevées sur les spectres UV-visible des différentes conditions

NL = Non Lyophilisée
L = Lyophilisée

*Conclusions*

Cette étape préliminaire montre que la lyophilisation de HbAm ne modifie pas la structure quaternaire de la molécule de façon significative. Cependant, la lyophilisation entraîne une oxydation partielle de l'hémoglobine. Les résultats montrent que les excipients testés permettent de protéger la molécule de l'oxydation induite par le procédé de lyophilisation sans en affecter sa structure quaternaire. Par ailleurs, parmi les excipients testés, le tréhalose et l'acide ascorbique protègent le mieux de l'oxydation.

Suivi de la stabilité de HbAm sur 5 jours

Cette étude est réalisée sur les échantillons reconstitués après lyophilisation avec et sans excipients et comparés à la molécule non lyophilisée.
Ceux-ci sont reconstitués à $T_0$ avec de l'eau ultrapure puis incubés à 37°C pour accélérer la dégradation de la molécule afin d'évaluer les effets des excipients choisis. Les analyses de structure, de fonctionnalité ainsi que le dosage de l'hémoglobine sont réalisés chaque jour ($J_0$, $J_1$, $J_2$, $J_3$, $J_4$).

*Suivi de la concentration en hémoglobine*

Les résultats montrent une diminution relative de la quantité d'hémoglobine. Cette diminution, caractéristique d'une dégradation ou adsorption de l'hémoglobine est observée pour la molécule non lyophilisée, ainsi que lyophilisée en présence d'acide ascorbique, de mannitol et de sucrose. Aucune diminution significative pour la molécule lyophilisée seule et en présence de tréhalose n'est observée.

*Suivi de la structure quaternaire*

Le pourcentage de pureté est mesuré à 280 nm au cours du temps. Le traitement des données (logiciel Graphpad®) permet de déterminer une courbe de tendance caractéristique de la cinétique de dégradation des molécules. Deux tendances se dessinent :

- Une régression linéaire pour l'ensemble des conditions excepté le tréhalose ;
- Un plateau suivi d'une phase de décroissance pour le tréhalose.

Les résultats montrent que l'acide ascorbique semble maintenir l'intégrité structurale de la molécule d'HbAm.
En effet :

- la molécule la moins stable est la molécule lyophilisée seule : $T_{1/2}$ = 2,9 jours et $k_d$ = 0.17j$^{-1}$; et
- la molécule lyophilisée en présence de tréhalose ou d'acide ascorbique est la plus stable avec $T_{1/2}$ = 5.4 et 4.7 jours respectivement, et $k_d$ = 0.12 et 0.10 j$^{-1}$ respectivement.

*Suivi de la fonctionnalité*

De même que pour le suivi de la structure, les données d'oxydation ont été analysées avec Graphpad® pour déterminer la cinétique d'oxydation des molécules.

L'évolution du pourcentage d'oxydation au cours du temps peut être représentée par une sigmoïde du type dose-réponse pour 3 conditions : Lyophilisée seule ; Lyophilisée avec tréhalose et sucrose.

L'aspect dose-réponse peut s'expliquer par une double cinétique ; la première correspondant à l'auto oxydation de la molécule : la transformation du fer ferreux en fer ferrique et la formation d'$O_2$ radicalaire ($O_2^{.-}$). L'$O_2$ radicalaire est une espèce oxydante qui catalyse la réaction d'oxydation (équation ci-dessous).

$$Hb (Fe^{2+}) + O_2 + H_2O \rightarrow HB (Fe^{3+}) OH_2 + O_2^{.-}$$

L'acide ascorbique et le tréhalose « captent » l'$O_2^{.-}$ évitant son interaction avec l'Hb : ceci explique le profil linéaire de la cinétique d'oxydation en présence de ces excipients. L'étude des spectres UV-visibles (résultats non présentés) montre un maintien de la fonctionnalité de l'hémoglobine jusqu'à J3 pour les échantillons composés d'acide ascorbique et jusqu'à J2 pour ceux contenant du tréhalose.

*Conclusions*

Durant cette étude à court terme, la prédominance de deux excipients a été observée pour le maintien de la structure, de la fonctionnalité et pour un ralentissement de l'oxydation de la molécule une fois reconstituée ; il s'agit du tréhalose et de l'acide ascorbique.

Evaluation de l'efficacité de HbAm

Les résultats montrent que, pour l'ensemble des conditions testées, comparé au contrôle, c'est-à-dire au milieu de préservation seul, les pourcentages de libération de LDH lui sont inférieurs.
Ceci montre que HbAm est efficace quelles que soient les conditions. Cependant, elle est plus ou moins efficace selon les excipients utilisés lors de la lyophilisation.

Une différence significative est notée entre la molécule non lyophilisée et la molécule lyophilisée en présence d'acide ascorbique, qui est la plus efficace. Enfin, la molécule lyophilisée semble plus efficace que la molécule non lyophilisée et que la molécule lyophilisée + tréhalose.

Etude de stabilité de HbAm à l'état lyophilisé

Les résultats présentés ici sont très préliminaires.

*Suivi de la concentration en hémoglobine*

L'évolution de la concentration au cours du temps dans les différentes conditions étudiées montre qu'il n'existe pas de différences significatives jusqu'à $J_2$. A partir de ce jour, la redispersion semble plus difficile.

*Suivi de la stabilité structurale*

Les résultats obtenus pour le suivi de la structure quaternaire de la molécule montrent des profils surprenants dans le cas de la molécule traitée par l'acide ascorbique.

Les tracés n'ont pas pu être intégrés à partir de $J_1$. En effet, le pic correspondant à l'hémoglobine est déstructuré.

Une comparaison entre la pureté de la molécule lyophilisée seule ou avec tréhalose a été effectuée.

Dans le cas de la molécule lyophilisée, la courbe décrit une tendance linéaire ($R^2$ = 0.9858). La molécule additionnée de tréhalose semble être plus stable. En effet le profil de la courbe montre une légère décroissance entre $J_0$ et $J_1$ puis une phase de stabilité jusqu'à $J_3$.

*Suivi de la stabilité fonctionnelle*

Au vu de l'évolution du pourcentage d'oxydation, l'acide ascorbique et le tréhalose semblent être aussi efficaces. Le premier décrit une évolution linéaire croissante tandis que le second présente une légère augmentation puis une phase de stabilité. Ces expériences devront être reproduites afin de confirmer ces premières observations.

**Résultats pour HbN**

L'ensemble des expériences réalisées dans le cadre du suivi de stabilité de HbAm est appliqué à HbN.

Suivi de la stabilité de HbN à $T_0$

*Suivi de la concentration en hémoglobine à $T_0$*

Les résultats du dosage d'hémoglobine après reconstitution de la molécule HbN dans les différentes conditions ne montrent aucune différence significative entre la molécule non lyophilisée, lyophilisée + tréhalose et lyophilisée + Mannitol. Cependant, une perte d'hémoglobine après lyophilisation de la molécule seule, additionnée de sucrose ou d'acide ascorbique, est notée.

Dans le cas présent, le mannitol comme le tréhalose, ont un effet positif sur la reconstitution.

*Suivi de la stabilité structurale de HbN après reconstitution*

Le pourcentage de pureté mesuré à $T_0$ à 280 nm (A) et 414 nm (B), ne montre, au vu des écarts-types, aucune différence significative à 280 nm, et ce quel que soit l'état de la molécule (lyophilisée ou non) et les excipients testés. Il en est de même pour le pourcentage de pureté à 414 nm. Ainsi, la lyophilisation ne semble pas induire de déstructuration de la molécule.

*Suivi de la fonctionnalité de la molécule HbN à T0*

Le pourcentage d'oxydation relatif obtenu après lyophilisation et calculé sur la base de la molécule non lyophilisée est mesuré.

Le suivi de l'état d'oxydation de la molécule à $T_0$ montre une oxydation de 35% pour la molécule lyophilisée seule soit 15 % supérieur à la molécule HbAm. L'ensemble des excipients testés offre à l'hémoglobine une protection contre l'oxydation. En effet, le pourcentage d'oxydation relevé à $T_0$ est compris entre 10 % pour le tréhalose et -20 % pour l'acide ascorbique qui est le plus protecteur. L'effet du tréhalose est moins efficace pour HbN que pour HbAm. Les spectres UV-visibles des différents échantillons présentent une superposition parfaite avec une signature spectrale d'une hémoglobine fonctionnelle.

Ainsi la fonctionnalité de la molécule HbN comme HbAm n'est pas affectée de façon significative par le processus de lyophilisation.

*Conclusions*

Au terme de cette étape, l'ensemble des excipients testés offre à la molécule un maintien de sa structure, de sa fonctionnalité et également une protection efficace contre l'oxydation particulièrement avec un traitement au sucrose ou à l'acide ascorbique. Contrairement à HbAm, le tréhalose apporte une protection contre l'oxydation moins efficace. En effet, dans le cas de HbN, l'oxydation est réduite (10 %) mais pas effacée. L'action de l'ensemble des excipients testés sera donc évaluée sur 5 jours afin d'en sélectionner les plus efficaces.

Suivi de la stabilité de HbN reconstituée sur 5 jours

*Suivi de la concentration en hémoglobine*

L'évolution de la concentration en hémoglobine au cours du temps montre que quelle que soit la condition considérée, la concentration en hémoglobine reste stable.

*Suivi de la structure quaternaire*

L'évolution du pourcentage de pureté à 280 nm montre que les différentes cinétiques adoptent une tendance linéaire (tableau 2).

Tableau 2 : Courbes de tendance d'HbN et constantes associées (NL = HbN Non Lyophilisée ; L = HbN Lyophilisée)

| Condition testée | Courbe de tendance | Equation | Constantes associées $R^2$ | | $k_d$ (j$^{-1}$) |
|---|---|---|---|---|---|
| | | | Demi-vie | $T_{1/2}$(j) | |
| NL | | | 0,9755 | 0.1643 $\pm$ 0.01502 | 3,043 |
| L | | | 0,9874 | 0.1714 $\pm$ 0.01119 | 2,917 |
| L +Tréhalose | | | 0,9737 | 0.1820 $\pm$ 0.01727 | 2,747 |
| L + Acide ascorbique | Régression linéaire | $\dfrac{\%\ puret\acute{e}\ (t)}{\%\ puret\acute{e}\ (t0)} = -k \times t$ | 0,9952 | 0.04049 $\pm$ 0.001995 | 12,348 |
| L + Mannitol | | | 0,9719 | 0.03847 $\pm$ 0.004627 | 12,997 |
| L + Sucrose | | | 0,9539 | 0.03423 $\pm$ 0.005324 | 14,607 |

Les données montrent un maintien de la structure protéique apporté par le sucrose ($T_{1/2}$ = 14.607 ; $k_d$ = 0,03423) le mannitol ($T_{1/2}$ = 12.997 ; $k_d$ = 0.03847) et l'acide ascorbique ($T_{1/2}$ = 12.348 ; $k_d$ = 0.0409). En ce qui concerne le tréhalose ($T_{1/2}$ = 2.747; $k_d$ = 0.1820), il se positionne au même niveau que les échantillons lyophilisés ou non.

*Suivi de la fonctionnalité*

Le suivi du pourcentage d'oxydation de l'hémoglobine révèle un effet protecteur apporté par le tréhalose jusqu' à J4 avec un pourcentage d'oxydation inférieur à 50%. Les autres excipients testés montrent un effet anti oxydant à T0 qu'ils perdent progressivement dans les jours qui suivent jusqu'à atteindre le niveau d'oxydation du contrôle non lyophilisé. Le profil des courbes adopte une tendance hyperbolique. L'oxydation de la molécule est alors très vite catalysée par l'oxygène radicalaire. L'étude des spectres UV-visibles obtenus pour les conditions testées montre un maintien de la fonctionnalité jusqu'à J3 lorsque l'hémoglobine est traitée au tréhalose (résultats non exposés ici).

*Conclusions*

Au terme de ce suivi sur une semaine, deux excipients se détachent : le tréhalose pour le maintien de la fonctionnalité et la protection efficace contre l'oxydation ; l'acide ascorbique pour le maintien de la structure de la molécule. Un autre excipient pourrait être conjugué au tréhalose, il s'agit du sucrose. Afin de compléter cette étude, les tests d'efficacité de la molécule formulée avec ces excipients seront réalisés.

Evaluation de l'efficacité de HbN

Le test d'efficacité de HbN est réalisé sur un modèle cellulaire. Les mesures effectuées concernent le pourcentage de viabilité (pourcentage de cellules vivantes) et la densité cellulaire.
Les résultats montrent que l'hémoglobine additionnée des différents excipients reste efficace sur les cellules. En effet, les cellules restent viables à plus de 95 % et la densité cellulaire est augmentée de façon significative par rapport au contrôle (X 1,7 en moyenne).
Une efficacité supérieure est observée pour la molécule lyophilisée en présence d'acide ascorbique ainsi que pour la molécule dans son état lyophilisé sans excipient.
Le tréhalose quant à lui décrit un profil semblable à celui de la molécule non lyophilisée.

Etude de stabilité de HbN à l'état lyophilisé

Pour cette étude, il est nécessaire de maintenir le produit lyophilisé en présence ou non d'excipients à 37°C afin d'accélérer la dégradation de la molécule. Ils sont ensuite reconstitués dans de l'eau afin de réaliser les analyses de suivi de structure, de fonction et de concentration.

*Suivi de la concentration en hémoglobine*

Le suivi de la concentration en hémoglobine après reconstitution montre le même profil déjà observé pour HbAm. En effet, aucune différence significative n'est observable jusqu'à J2 avant de débuter une phase de décroissance synonyme d'une redispersion difficile.

*Suivi de la structure quaternaire*

De même que pour HbAm, les profils des chromatogrammes sont surprenants pour l'acide ascorbique en particulier avec une déstructuration du pic caractéristique de l'hémoglobine.

*Suivi de la fonctionnalité*

Dans le cas de HbN, la molécule lyophilisée seule sans excipient est très fortement oxydée. Lorsque l'effet des excipients choisis est étudié, le tréhalose apporte une protection contre l'oxydation qui est stable de J 1 à J3. Il en est de même pour l'acide ascorbique avec un effet protecteur supérieur. Le profil obtenu avec une légère augmentation puis une phase de stabilité est semblable à celui de HbAm. Il existe malgré tout une différence significative entre les deux molécules au niveau du pourcentage d'oxydation initial et final, deux fois plus élevé pour HbN.

## Exemple 2

- Des échantillons de 20ml d'HbAm de composition suivante ont été lyophilisés:

    - Echantillon A (témoin) : HbAm dans de l'eau ultrapure ;
    - Echantillon B : HbAm dans une solution aqueuse à 5 mM d'acide ascorbique ;
    - Echantillon C : HbAm dans une solution aqueuse comprenant 55 mg/ml de tréhalose ;
    - Echantillon D : HbAm dans une solution aqueuse comprenant 55 mg/ml de tréhalose et 5 mM d'acide ascorbique.

Ces échantillons lyophilisés ont ensuite été redispersés dans i) de l'eau ultrapure, ii) une solution aqueuse à 5 mM d'acide ascorbique, iii) une solution de stabilisation (= solution aqueuse comprenant 90 mM de NaCl, 23 Mm de Na-gluconate, 2,5 mM de $CaCl_2$, 27 mM de Na-acétate, 1,5 mM de $MgCl_2$, 5 mM de KCl, pH de 7,1 $\pm$ 0,5) ou iv) la solution iii) comprenant en outre 5 mM d'acide ascorbique.

La fonctionnalité des lyophilisats redispersés obtenus a été mesurée comme indiqué en exemple 1.

Les résultats montrent que la présence de tréhalose lors de la formulation limite la dégradation de HbAm. Le pourcentage d'hémoglobine dans les échantillons C et D (i.e. avec tréhalose) redispersés, aux alentours de 88%, est significativement supérieur à celui des échantillons A et B, aux alentours de 82-84%.

En outre, la présence d'acide ascorbique lors de la formulation limite l'oxydation liée à la lyophilisation.

- Des expériences de lyophilisation ont été réalisées avec un échantillon E. Cet échantillon E comprend HbAm dans une solution aqueuse comprenant 23 Mm de Na-gluconate, 2,5 mM de $CaCl_2$, 27 mM de Na-acétate, 1,5 mM de $MgCl_2$, 5 mM de KCl, pH de 7,1 $\pm$ 0,5, 65 mg/ml de tréhalose et 5 mM d'acide ascorbique.

La lyophilisation a pu être effectuée de façon très satisfaisante.

## Exemple 3

Matériel :

Appareil de lyophilisation de laboratoire LABCONCO

Méthodes :

Aliquots de 1ml de HbAm (M101) ou HbN (M201)

Les échantillons de 1 ml sont congelés avant lyophilisation et répartis dans des vials comme indiqué dans le Tableau 1 ci-dessous :

| Référence | Contenu | T de congélation |
|---|---|---|
| A | M101 | -20°C |
| B | M101 | -80°C |
| C | M201 | -20°C |
| D | M201 | -80°C |
| E | 5% Mannitol | -20°C |
| F | 5% Mannitol | -80°C |
| Tableau 1 : Résumé des conditions testées. Les conditions E et F sont des contrôles positifs de lyophilisation | | |

Une fois congelés, les vials sont placés dans le lyophilisateur.

Résultats :

*Résultats de la lyophilisation :*

Après vérification, le congélateur -20°C montrait une température effective de -17°C. Après chargement, à la mise sous pression de l'appareil, les échantillons A et B sortent en mousse du vial. L'échantillon C montre une fusion partielle limitée. Les autres échantillons étant dans la zone « glace » du diagramme des tensions vapeurs, restent stables. Après 30 minutes, les échantillons D, E et F commencent une sublimation apparemment sous de bonnes conditions mais hors de contrôle. Après 6 heures de fonctionnement, l'essai est arrêté. Les échantillons D, E et F se présentent sous une belle pastille avec une cristallisation nettement apparente. L'échantillon D présente une surface brillante laissant présager la formation d'une peau due à la congélation. La redispersion se fait en quelques minutes, mais pour l'échantillon E présence de quelques « grumeaux ».

*Résultats des analyses HPLC :*

Des analyses HPLC ont été réalisées afin de vérifier la pureté et la structure des hémoglobines post-lyophilisation, et afin de vérifier si celles-ci n'ont pas été dégradées au cours de la lyophilisation.

| Echantillons | % Pureté (%) | Agglomérats (%) | Dissociation (%) |
|---|---|---|---|
| M101 Avant Lyoph | 91.80 | 4.01 | 1.49 |
| M101 Post Lyoph | 91.69 | 3.15 | 1.27 |
| | | | |
| M201 Avant Lyoph | 86.55 | 5.28 | 2.81 |
| M201 Post Lyoph | 82.32 | 5.60 | 4.39 |

*Résultats des dosages Hb :*

Des dosages de l'hémoglobine ont été réalisés avant et après lyophilisation, et une fois redispersée.

| Echantillons | Molécules | Avant lyophilisation (mg/ml) | Après lyophilisation (mg/ml) |
|---|---|---|---|
| E | M201 | 46.83 | 40.06 |
| F | M101 | 49.05 | 41.87 |

*Conclusion :*

La première conclusion est que les 2 molécules sont lyophilisables, puisqu'il a été obtenu de belles pastilles pour les conditions à -80°C. En effet la congélation à -20°C conduit à la fusion des molécules lors de la mise sous vide. La sublimation se passe bien mais compte tenu de la durée de la manipulation, il n'y a pratiquement pas eu de dessiccation secondaire, qui, prolongée, ne pourrait qu'améliorer en particulier les conditions d'humidité et de redispersion. Les résultats des analyses HPLC montrent que la molécule M201 subit une dégradation partielle (~4%) lors de la lyophilisation. En ce qui concerne la molécule M101, elle est stable.

**Revendications**

1.  Lyophilisat comprenant au moins une hémoglobine extracellulaire de la famille des *Arenicolidae* ou de la famille des *Nereididae*, et un agent stabilisant choisi parmi les disaccharides.

2.  Lyophilisat selon la revendication 1, dans lequel l'hémoglobine extracellulaire est choisie parmi l'hémoglobine extracellulaire d'*Arenicola marina* et l'hémoglobine extracellulaire de *Nereis.*

3.  Lyophilisat selon l'une des revendications 1 ou 2, dans lequel le disaccharide est choisi parmi le tréhalose et le saccharose.

4.  Lyophilisat selon l'une des revendications 1 à 3, dans lequel l'agent stabilisant comprend également un polyol et un antioxydant, de préférence le polyol est choisi parmi le mannitol et le sorbitol ; et de préférence l'antioxydant est l'acide ascorbique.

5.  Procédé de préparation d'un lyophilisat selon l'une des revendications 1 à 4, comprenant :

    i) le mélange d'une solution comprenant au moins une hémoglobine extracellulaire de la famille des *Arenicolidae* ou de la famille des *Nereididae* avec un agent stabilisant choisi parmi les disaccharides,
    ii) la congélation du mélange obtenu en i) à une température comprise entre -10°C et -100°C, de préférence entre -20°C et -100°C, pendant un temps d'au moins 24h ;
    iii) la sublimation du mélange congelé obtenu en ii) pendant au moins 2h, sous vide ;
    iv) le séchage final du mélange obtenu en iii), jusqu'à l'obtention d'une poudre.

6.  Procédé selon la revendication 5, dans lequel l'agent stabilisant choisi parmi les disaccharides est présent dans le mélange de l'étape i) en une concentration comprise entre 1 et 500 mg/ml.

7.  Procédé selon la revendication 5, dans lequel l'agent stabilisant comprend également un polyol et un antioxydant, l'antioxydant étant présent dans le mélange de l'étape i) en une concentration comprise entre 3 et 20 mM.

8.  Procédé selon la revendication 5 à 7, dans lequel le disaccharide présent dans le mélange de l'étape i) est choisi parmi le tréhalose ou le saccharose présent en une concentration comprise entre 50 et 70 mg/ml, de préférence d'environ 55 mg/ml ou 65 mg/ml, le tréhalose ou le saccharose présent en une concentration d'environ 100 mg/ml.

9.  Procédé selon l'une des revendications 5 à 8, dans lequel la congélation de l'étape ii) est effectuée à une température comprise entre -10°C et -90°C, de préférence entre -20°C et - 90°C, pendant au moins 24h.

10. Procédé selon l'une des revendications 5 à 9, dans lequel la sublimation de l'étape iii) est effectuée pendant au moins 4h.

**Patentansprüche**

1.  Lyophilisat umfassend mindestens ein extrazelluläres Hämoglobin der Familie der Arenicolidae oder der Familie der Nereiden und ein Stabilisierungsmittel, ausgewählt aus den Disacchariden.

2.  Lyophilisat nach Anspruch 1, bei dem das extrazelluläre Hämoglobin ausgewählt ist aus dem extrazellulären Hämoglobin der Arenicola marina und dem extrazellulären Hämoglobin der Nereis.

3.  Lyophilisat nach einem der Anspruch 1 oder 2, bei dem das Disaccharid ausgewählt ist aus Trehalose und Saccharose.

**4.** Lyophilisat nach einem der Anspruch 1 bis 3, bei dem das Stabilisierungsmittel gleichfalls ein Polyol und ein Antioxidans umfasst, wobei das Polyol vorzugsweise ausgewählt ist aus dem Mannitol und dem Sorbitol; und vorzugsweise das Antioxidans Ascorbinsäure ist.

**5.** Verfahren zur Herstellung eines Lyophilisats nach einem der Ansprüche 1 bis 4, umfassend:

> i) die Mischung einer Lösung, umfassend mindestens ein extrazelluläres Hämoglobin der Familie der Arenicolidae oder der Familie der Nereiden mit einem Stabilisierungsmittel, ausgewählt aus den Disacchariden,
> ii) das Einfrieren der mit i) erhaltenen Mischung bei einer Temperatur zwischen -10°C und -100°C, vorzugsweise zwischen -20°C und -100°C, während einer Zeit von mindestens 24 h;
> iii) das Sublimieren der mit ii) erhaltenen Mischung während mindestens 2h unter Vakuum;
> iv) die endgültige Trocknung der mit iii) erhaltenen Mischung bis zum Erhalten eines Pulvers.

**6.** Verfahren nach Anspruch 5, bei dem das aus den Disacchariden ausgewählte Stabilisierungsmittel in der Mischung des Schritts i) bei einer Konzentration zwischen 1 und 500 mg/ml vorhanden ist.

**7.** Verfahren nach Anspruch 5, bei dem das Stabilisierungsmittel gleichfalls ein Polyol und ein Antioxidans umfasst, wobei das Antioxidans in der Mischung des Schritts i) in einer Konzentration zwischen 3 und 20 mM vorhanden ist.

**8.** Verfahren nach Anspruch 5 bis 7, bei dem das in der Mischung des Schrittes i) vorhandenen Disaccharid ausgewählt ist aus der Trehalose oder der Saccharose, vorhanden in einer Konzentration zwischen 50 und 70 mg/ml, vorzugsweise ungefähr 55 mg/ml oder 65 mg/ml, der Trehalose oder der Saccharose, vorhanden in einer Konzentration von ungefähr 100 mg/ml.

**9.** Verfahren nach einem der Ansprüche 5 bis 8, bei dem das Einfrieren des Schrittes ii) bei einer Temperatur zwischen -10°C und -90°C, vorzugsweise zwischen -20°C und -90°C während mindestens 24h durchgeführt wird.

**10.** Verfahren nach einem der Ansprüche 5 bis 9, bei dem die Sublimierung des Schrittes iii) bei mindestens 4h durchgeführt wird.

**Claims**

**1.** Lyophilizate comprising at least one extracellular hemoglobin of the family *Arenicolidae* or the family *Nereididae*, and a stabilizer chosen from disaccharides.

**2.** Lyophilizate according to claim 1, wherein the extracellular hemoglobin is chosen from the extracellular hemoglobin of *Arenicola marina* and the extracellular hemoglobin of *Nereis.*

**3.** Lyophilizate according to any one of claims 1 or 2, wherein the disaccharide is chosen from trehalose and sucrose.

**4.** Lyophilizate according to any one of claims 1 to 3, wherein the stabilizer further comprises a polyol and an antioxidant, preferably the polyol is chosen from mannitol and sorbitol; and preferably the antoioxidant is ascorbic acid.

**5.** A process for preparing a lyophilizate as claimed any one of claims 1 to 4, comprising:

> i) the mixing of a solution comprising at least one extracellular hemoglobin of the family *Arenicolidae* or of the family *Nereididae* with a stabilizer chosen from disaccharides,
> ii) the freezing of the mixture obtained in i) at a temperature of between -10°C and -100°C, preferably between -20°C and -100°C, for a time of at least 24 h;
> iii) the sublimation of the frozen mixture obtained in ii) for at least 2 h, under vacuum;
> iv) the final drying of the mixture obtained in iii), until a powder is obtained.

**6.** The process according to claim 5, wherein the stabilizer chosen from disaccharides is present in the mixture of step i) in a concentration of between 1 and 500 mg/ml.

**7.** The process according to claim 5, wherein the stabilizer further comprises a polyol and an antioxidant, the antioxidant being present in the mixture of step i) in a concentration of between 3 and 20 mM.

8. The process according to any one of claims 5 to 7, wherein the disaccharide present in the mixture of step i) is chosen from trehalose or sucrose present in a concentration of between 50 and 70 mg/ml, preferably of approximately 55 mg/ml or 65 mg/ml, trehalose or sucrose present in a concentration of approximately 100 mg/ml.

9. The process according to any one of claims 5 to 8, wherein the freezing of step ii) is carried out at a temperature of between -10°C and - 90°C, preferably between -20°C and -90°C, for at least 24 h.

10. The process according to any one of claims 5 to 9, wherein the sublimation of step iii) is carried out for at least 4 h.

**Figure 1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010128159 A
- WO 2007035583 A
- WO 03026786 A

**Littérature non-brevet citée dans la description**

- **HELLER.MARTIN.C ; CARPENTER.JOHN.F ; RANDOLPH.THEODORE.W.** Protein Formulation and Lyophilization Cycle Design : Prevention of Damage due to Freeze-Concentration Induced Phase Separation. *Biotechnology and Bioengineering,* 1999, vol. 63 (2